# EUROPEAN PATENT SPECIFICATION

(11) **EP 2 718 400 B1**
(45) Date of publication and mention of the grant of the patent: **10.04.2019**
(21) Application number: 12723745.1
(22) Date of filing: 17.04.2012
(51) Int. Cl.: C10B 57/00, G01N 25/16

(54) **THE METHOD OF A COAL OR COAL BLEND EXPANSION PRESSURE DETERMINATION AND THE DEVICE FOR THE METHOD**
VERFAHREN ZUR BESTIMMUNG DES EXPANSIONSDRUCKS VON KOHLE ODER EINER KOHLEMISCHUNG UND VORRICHTUNG FÜR DAS VERFAHREN
PROCÉDÉ DE DÉTERMINATION DE PRESSION D'EXPANSION D'UN CHARBON OU D'UN MÉLANGE À BASE DE CHARBON ET DISPOSITIF POUR LE PROCÉDÉ

(30) Priority: 06.06.2011 PL 39514611
(43) Date of publication of application: 16.04.2014
(73) Proprietor: Instytut Chemicznej Przeróbki Wegla, 41-803 Zabrze (PL)
(72) Inventor: SCIAZKO, Marek, PL-40-558 Katowice (PL); SOBOLEWSKI, Aleksander, PL-41-800 Zabrze (PL); MERTAS, Bartosz, PL-41-800 Zabrze (PL)
(74) Representative: Korga, Leokadia
(86) International application number: PCT/IB2012/051911
(87) International publication number: WO 2012/168802

(56) References cited:
- JP-A- 4 272 992
- JP-A- H03 188 351
- JP-A- 2008 143 928
- JP-A- 2009 144 001
- KR-A- 20040 106 183

## Description

The subject of the invention is the method of a coal or coal blend expansion pressure determination and the subsequent appropriate device for the method.

Expansion pressure is a significant phenomenon that occurs during the coking process and has an impact on the safety of the coke oven battery walls as well as on the quality of the produced coke. Charged coal blend into the coking chamber and indirectly heated is subject to carbonisation, and in the specified temperature range, it may soften and, exert considerable pressure on the chamber walls. This may lead to damage or, in extreme cases, to the destruction of the chamber walls. When planning the coking process, it is essential to be aware of the value of the coal charge expansion pressure, where a high value indicates the possibility of high-quality blast furnace coke production. However, the safety of the ceramic block of the coking chamber may be endangered by this production, and in extreme conditions, there is potential for its destruction.

There are a few known methods of measuring and forecasting the value of the expansion pressure that use direct and indirect measurements.
A muffle oven and a steel box are used in the muffle assessment test to determine the coal expansion pressure; this test is commonly used in the literature. In this test, the coal sample is compacted in a steel box, which is subsequently placed into a muffle oven and heated to 900°C. The level of pressure is estimated in the expansion test based on the distortion of the steel box walls and the appearance of the coke after the sample has been removed from the oven and the steel box has been cooled down with water.

Another laboratory device that is used for the normalised expansion pressure measurement is the Polish Standard PN/G-04522. This device consists of an electric oven with temperature adjustment via a resistor, a crucible with a replaceable perforated bottom, a thermoelement with a temperature gauge and a small piston connected to a mercury pressure gauge. Determining the expansion pressure using this device consists of measuring the force with the mercury pressure gauge, which is located directly on the small piston that is placed on the coal sample, and the expansion pressure is calculated with the appropriate formula. In this method, an 80 g sample of coal with a size below 1.4 mm is compacted in the crucible in a dry-air condition. Then, the oven is heated to 250°C, and the crucible containing the sample is entered into the oven and heated at a rate of 10°C/min. The oven heating elements are located in the bottom part of the oven, the thermoelement rests on the bottom of crucible, and the crucible with the sample is heated from the bottom. Pressure gauge readings, which determine the force acting on the small piston, are read at specified intervals. The expansion pressure is calculated based on the maximum force acting on the small piston.

Another well-known solution is called a test stand the method of investigating the expansion pressure phenomenon. In this procedure the expansion pressure is determined by placing the 50 - 100 g coal sample, with a size composition of 1-3 mm, in a steel sleeve and inserting the sleeve into the oven. The sample then is heated in a circumferential manner around the sleeve. The sample temperature and pressure acting on the small piston are measured during each test.

Another group of assessment methods for the determination of the expansion pressure are called indirect methods. In these methods, a physical value is measured and then correlated with the expansion pressure.

The device from the Spanish patent description No. 524,258 of the INCAR CSIC Institute allows the expansion and contraction of the coals or the coal blend to be measured. Using this device, comparative tests are conducted; the expansion pressure is measured in the oven using a movable wall. The readings from these measurements show that the coal samples with a contraction of at least 10 mm are safe in terms of the expansion pressure. From JP2008 143928 patent is known device for continuously measuring the coal expansion pressure by a load cell comprising a heating oven having a measuring space having an upper part opening in its center and a heating body at its bottom part, a heat-insulating sleeve loaded on the bottom part in the measuring space and housing the coal to be measured, a heat-insulating lid body covering the heat-insulating sleeve and a piston of which upper end part is fixed at an upper part structural frame of the heating oven through a load cell and having a heat-insulating material kept on the heat-insulating lid body at its lower end part , and further, if necessary, the above piston includes a counter weight corresponding to the weights of the piston and heat-insulating material at its upper part.

From KR 20040106183 patent application is known an apparatus for measuring expansion and contraction behavior of coal generated during coke production, wherein expansion and contraction behavior of coal generated during coal carbonization is measured. In an apparatus for measuring expansion and contraction behavior of coal charged as coal carbonization is being proceeded in an one-way heating and carbonization furnace, the apparatus for measuring expansion and contraction behavior of coal generated during coal carbonization comprises an insulating plate installed on a surface of coal charged into an oven chamber of the carbonization furnace in such a manner that the insulating plate is integrally moved together with expansion and contraction behavior of coal generated as carbonization is being proceeded; a ceramic tube one end of which is connected to an upper part of the insulating plate, and other end of which is projected to the outside by passing through hatch of an outer case of the carbonization furnace; and a displacement measuring sensor attached to an outward projected one end of the ceramic tube to generate electrical signals from displacement according to the measured movements of the insulating plate and the ceramic tube by measuring movements of the insulating plate and the ceramic tube.

From JP2009144001 is known the apparatus which is equipped with an outer tube having a coal sample room installed in the center inside of the longitudinal direction of the apparatus main body, a pair of upper-lower side mesh plates arranged in the upper and lower sides of the coal sample room of the outer tube, a pair of upper-lower side mesh plate fixing tubes that push a pair of the mesh plates from the upper part and the lower part in the outer tube to fix them, and a gas introducing port and a gas exhausting port that are for passing the gas into the coal sample room. The apparatus is also constituted by a pair of upper-lower side caps which, while sealing the edge parts of the upper and lower side outer tubes and the edge parts of the mesh plate fixing tubes through packings, serve to fix them from the periphery of the outer tube.

Whereas from JP H03188351 patent it was found apparatus to detect expansibility of coal under the same state as the state when the coal is undergoes dry distillation in a coke furnace by mounting a permeable material on the upper surface of a sample which is inserted into a metal container, compressing the sample, and exhausting the gas which is generated by the softening and melting of the sample by heating through the permeable material. A permeable material is arranged on the upper surface of a sample. The sample is compressed with a piston in a metal container. The metal container is heated in an electric furnace at a constant temperature increase rate. The gas and a part of the liquid material which are generated by the softening and the melting of the sample by heating are discharged from the metal container through the permeable material which is mounted on the upper surface of the sample. Therefore, the sample is expanded and contracted in the same way as in the case when the sample undergoes dry distillation in an actual coke furnace in the metal container. Vertical displacement of the piston caused by the expansion and contraction is measured.

These methods in real conditions does not provide direct information regarding the expansion pressure, but provide information regarding the potential propensity of charge contraction in the chamber, which causes so-called "heavy runs" in the coking chamber.

A different solution for the determination of the expansion pressure is the measurement of the internal gas pressure, which is monitored by probes located inside the coal charge. The value of the gas pressure being measured is often linked to the value of the expansion pressure; however, there are no unequivocal results confirming the occurrence of such a relationship.

At present, the most reliable method for determining the expansion pressure is the use of a movable wall oven. This allows coking test to be conducted with a coal charge of approximately 300-500 kg, depending on the construction of the oven. One of the oven's walls is linked to the force sensor, which allows a continuous measurement of the force acting on this wall. This force is then automatically recalculated into an expansion pressure. The advantage of the use of such a device is the high reliability of the results obtained. However, this type of testing is highly expensive, which results from the necessity of employing several people for oven operation, the amount of the coal charge and the high cost of electricity necessary to heat this charge to a temperature over 1000°C for 12 or more hours.

The current methods for measuring the expansion pressure usually do not produce comparable results, which are measured in the movable wall oven with the value of this parameter. This lack of comparable results is primarily caused by the following: 1) only a visual assessment of the effect of the pressure action is possible; 2) the measurements include not only of the expansion pressure but also the pressure of the released gases; or 3) the measurement is a result of the substitute parameter and is only linked to the expansion pressure, as seen in the case of the contraction measurement method described in described patents.

The objective of the method, according to this invention, is to determine, in a reliable way, the values of the coal and the coal blend expansion pressure in laboratory conditions by simulating the behaviour of the coal throughout the industrial coking process in the coking chamber.

In order to achieve this objective, the invention provides a method according to claim 1.

Favourably, when the inert material is either anthracite or coke breeze.

Favourably, when the size composition of the inert material is 1.0 - 1.5 mm.

Favourably, when the size composition of the coal sample is ≤5 mm.

Favourably, when the rate of heating is 3 K/min.

Favourably, when the thermoelement is located at the mid-height of the sample.

Furthermore the invention provides a device according to claim 7.

The determination of the expansion pressure proceeds favourably under the following conditions:
Favourably, when the holes of the sleeve are of 1 mm diameter.

Favourably, when the guard of temperature measurement unit is located at the mid-height of the sleeve.

The essential advantage of this solution, according to the invention, is that the coal or the coal blend is subjected to heating in the perforated sleeve which is located inside the crucible. This allows the volatile matter being released to flow through it. In addition, the granular material placed outside the perforated sleeve prevents the volatile matter from travelling through the bed, creating a resistance for the gases. This, in turn, makes it possible to simulate the gas evacuation path through the semi-coke and coke areas in the coking chamber. As a result of such an arrangement, the force being measured through the piston with the force measurement system has its source in the expansion of the sample and is linked to the swelling of the individual grains and only partially pressure of the gas being released.

An additional and vital advantage of the application of this method is the fact that the obtained results from the expansion pressure measurements can be quantitatively linked to the value of the expansion pressure being measured in the movable wall oven with a linear function.

Upon the application of this method for measuring the expansion pressure, according to the invention, we can create conditions similar to real industrial conditions and improve the accuracy of the experiments performed, which is not possible with the other known design solutions or with other methods of measuring the expansion pressure.

This novel method for determining the expansion pressure and its execution are demonstrated in the following figures:

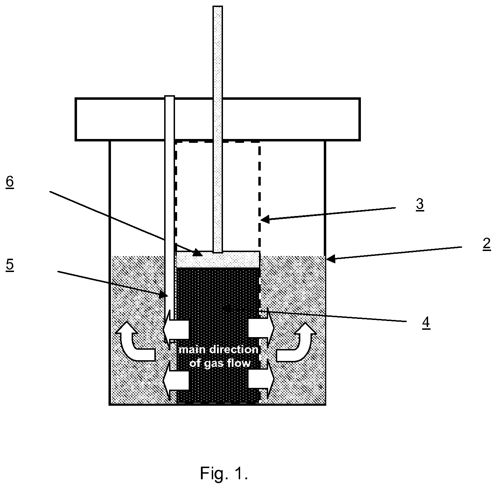

Fig. 1 - Crucible showing a schematic of the direction of the gas flow that does not generate expansion pressure;
Fig. 2 - Diagram of the device for testing the expansion pressure.

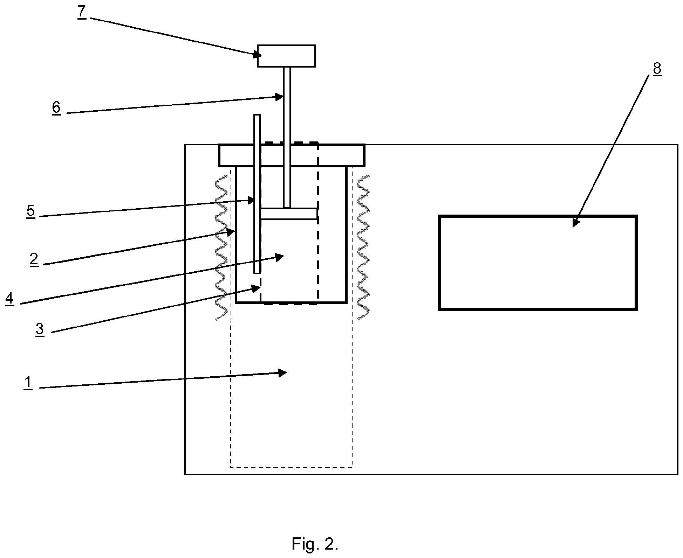

The device, according to the diagram shown in Fig. 2, consists of an electrically heated oven 1 where the crucible 2 is located in. Inside the crucible 2 is a perforated sleeve 3 with holes 1 mm in diameter. There is a guard of the thermocouple 5 at the mid-height of the sleeve 3. In the sleeve 3, there is a coal sample 4 that is held down by a the transmitting force piston 6 laying directly on the sample surface that is in direct contact to the force measurement system 7. The space between the internal wall of the crucible 2 and the sleeve 3 is filled in with coke breeze or anthracite 1-1.5 mm in size. The sample of coal/coal blend 4 with a size ≤5 mm is placed inside of the sleeve 3, and the piston 6 is attached. The piston 6 is in direct contact to the force measurement system 7. Next, the heating of the oven 1 is started, and the control system 8 adjusts the heating to a rate of 3 K/min. The registration and archiving system 8 reads and continuously saves the data taken from the thermoelement, located in the guard 5, and the force measurement system 7. The measurement is considered to have been completed after the temperature has reached 650°C.

## Claims

1. Method of determination of the expansion pressure of coal or a coal blend in a laboratory test, wherein:
- a coal or coal blend sample is subjected to heating in a crucible which is located in an electrically heated oven;
- said sample is located in a perforated sleeve which is inside said crucible;
- between the inner wall of the crucible and the sleeve, there is an inert granular material that is greater in size than the holes in the sleeve;
- said crucible is heated around its circumference at a rate of several degrees per minute from the ambient temperature to a temperature greater than the end of coal plasticity, the temperature being measured with a thermocouple placed on the sleeve wall;
- a piston located on the top of the sample transmits the pressure due to the expansion of said sample to a force measurement system;
- the expansion pressure of said sample is determined on the basis of the force measured by said force measuring system.

2. The method according to claim 1 **is characterised in that** the inert material is anthracite or coke breeze.

3. The method according to claim 1 **is characterised in that** the inert material size distribution is 1.0 - 1.5 mm.

4. The method according to claim 1 **is characterised in that** the coal/coal blend sample size is ≤5 mm.

5. The method according to claim 1 **is characterised in that** the heating rate is 3 K/min.

6. The method according to claim 1 **is characterised in that** the temperature measurement is conducted at the mid-height of the sample.

7. Device for carrying out a method of determination of the expansion pressure of coal or a coal blend comprising:
- an electrically heated oven;
- a crucible located inside the electrically heated oven;
- a thermocouple;
- a force measuring system;
- a perforated sleeve located inside the crucible and having a guard for said thermocouple, wherein the guard is located on the surface of said perforated sleeve and wherein the perforated sleeve is adapted to receive a coal or coal blend sample;
- between the inner wall of the crucible and the sleeve, an inert granular material that is greater in size than the holes in the sleeve;
- a piston arranged to hold down said sample and placed adjacent a force measurement system, such that the piston transmits the pressure due to the expansion of said sample to said force measurement system;
- a control system configured to perform heating control and configured to acquire data from the thermocouple and the force measurement system.

8. The device according to claim 7 **is characterised in that** diameter of the sleeve (3) holes is 1 mm.

9. The device according to claim 7 **is characterised in that** the guard of the thermocouple (5) is located at the mid-height of the sleeve (3).

## Patentansprüche

1. Das Verfahren zur Bestimmung des Wertes des Expansionsdrucks von Kohle oder Kohlegemischen durch Durchführen eines Tests im Labormaßstab, **dadurch gekennzeichnet, dass**:
- eine Probe von Kohle oder Kohlegemisch in einem Tiegel erhitzt wird, der sich in einem Elektroofen befindet
- diese Probe sich innerhalb einer perforierten Hülse befindet, die sich in diesem Tiegel befindet
- zwischen der Tiegelinnenwand und der äußeren Hülsenwand sich ein inertes körniges Material mit einer Korngröße befindet, die größer ist als Löcher in der Hülse,
- dieser Tiegel am Umfang mit einer Geschwindigkeit von mehreren Grad pro Minute erhitzt wird, ab der Umgebungstemperatur bis auf eine Temperatur oberhalb der Endplastizität von Kohle, wobei die Temperatur mittels eines an der Wand der Hülse befindlichen Thermoelements gemessen wird;
- ein Kolben, der sich auf der Oberseite der Probe befindet, den Druck, der sich aus der Ausdehnung der Probe ergibt, an das Kraftmesssystem überträgt,
- der Expansionsdruck der Probe anhand der vom Messsystem gemessenen Kraft bestimmt wird.

2. Das Verfahren zum Bestimmen eines Druckwertes nach Anspruch 1, **dadurch gekennzeichnet, dass** das inerte Material Anthrazit oder Koksgrus ist.

3. Das Verfahren zur Bestimmung eines Druckwertes nach Anspruch 1, **dadurch gekennzeichnet, dass** die Korngröße des inerten Materials 1,0 - 1,5 mm beträgt.

4. Das Verfahren zur Bestimmung eines Druckwertes nach Anspruch 1, **dadurch gekennzeichnet, dass** die Korngröße der Kohleprobe ≤ 5 mm ist.

5. Das Verfahren zur Bestimmung eines Druckwertes nach Anspruch 1, **dadurch gekennzeichnet, dass** die Aufheizgeschwindigkeit 3 K/min beträgt.

6. Das Verfahren zur Bestimmung eines Druckwertes nach Anspruch 1, **dadurch gekennzeichnet, dass** die Temperaturmessung auf halber Höhe der Probe durchgeführt wird.

7. Die Vorrichtung zur Bestimmung des Expansionsdrucks von Kohle oder Kohlegemisch besteht aus:
- einem elektrisch beheizten Ofen;
- einem Tiegel in einem elektrisch beheizten Ofen angeordneten;
- einem Thermoelement;
- einem Kraftmesssystem;
- einer perforierten Hülse, die sich im Tiegel befindet, wo die Thermoelementhülle angeordnet ist, wobei diese Hülle sich an der Wand der Hülse befindet und die perforierte Hülse das Einführen in diese Kohle oder Kohlegemisch ermöglicht;
- zwischen der Tiegelinnenwand und der äußeren Hülsenwand befindet sich ein inertes körniges Material mit einer Korngröße, die größer ist als die Löcher in der Hülse;
- einem Kolben, der so positioniert ist, dass er gegen die Oberseite der Probenoberfläche drückt und das Kraftmesssystem derartig berührt, dass der Kolben aufgrund der Expansion der Probe einen Druck auf das Kraftmesssystem überträgt;
- eine Steuerung, die so eingerichtet ist, dass sie die Heizung steuert und Daten vom Thermoelement und Kraftmesssystem erfasst.

8. Die Vorrichtung nach Anspruch 7, **dadurch gekennzeichnet, dass** die Löcher in der Hülse (3) einen Durchmesser von 1 mm haben.

9. Die Vorrichtung nach Anspruch 7 **dadurch gekennzeichnet, dass** die Hülle des Temperaturmesselements (5) auf halber Höhe der Hülse (3) angeordnet ist.

## Revendications

1. La manière de déterminer la valeur de pression de détente de charbon ou de mélanges charbonneux à l'aide d'un test exécuté à l'échelle du laboratoire, **caractérise par** ce que:
- L'échantillon de charbon ou de mélange charbonneux est soumis au réchauffement dans un creuset qui se trouve au four électrique
- Cet échantillon se trouve à l'intérieur d'une douille perforée qui se trouve dans ce creuset
- Entre la paroi intérieure du creuset et la paroi extérieure de la douille se trouve du matériau granulaire inerte, d'une granulométrie plus grande que les orifices dans la douille,
- Ce creuset est réchauffé sur sa périphérie à la vitesse de quelques degrés par minute, à partir de la température ambiante jusqu'à la température au-dessus de celle de la plasticité de charbon, néanmoins la température est mesurée avec un thermo élément situé sur la paroi de la douille
- Le piston situé sur la surface supérieure de l'échantillon transmet la pression résultant de la dilatation de l'échantillon vers le système de mesure de la force,
- La pression de dilatation de l'échantillon est déterminée sur la base de la force mesurée par le système de mesure de la force.

2. La manière de déterminer la valeur de pression selon la revendication 1 **se caractérise par** ce que le matériau inerte est l'anthracite ou le coke grésillon.

3. La manière de déterminer la valeur de pression selon la revendication 1 **se caractérise par** ce que la granulométrie du matériau inerte est de 1,0 - 1,5 mm.

4. La manière de déterminer la valeur de pression selon la revendication 1 **se caractérise par** ce que la granulométrie de l'échantillon de charbon est ≤5 mm

5. La manière de déterminer la valeur de pression selon la revendication 1 **se caractérise par** ce que la vitesse de réchauffement est de 3 K/min.

6. La manière de déterminer la valeur de pression selon la revendication 1 **se caractérise par** ce que la mesure de température est effectuée à la mi-hauteur de l'échantillon.

7. L'appareil pour déterminer la pression de détente de charbon ou de mélange charbonneux est composé de:
- four électriquement réchauffé;
- creuset situé à l'intérieure du four électriquement réchauffé;
- thermo élément;
- système de mesure de force;
- douille perforée placée à l'intérieur du creuset où se trouve aussi la protection du thermo élément, celle-ci se trouve sur la paroi de la douille et la douille perforée rend possible d'y introduire un échantillon de charbon ou de mélange charbonneux;
- Entre la paroi intérieure du creuset et extérieure de la douille se trouve du matériau granulaire inerte, d'une granulométrie supérieure aux orifices pratiqués dans le creuset;
- Le piston est situé de telle manière qu'il presse du dessus la surface de l'échantillon et il y a du contact avec le système de mesure de la force de sorte que le piston transmet la pression à cause de la dilatation de l'échantillon vers le système de mesure de la force;
- Le système de commande est réglé de sorte qu'il contrôle le réchauffement et recueille les données provenant de la thermo pair et du système de mesure de la force.

8. L'appareil selon la revendication 7 **se caractérise par** ce que les orifices dans la douille (3) ont 1 mm de diamètre.

9. L'appareil selon la revendication 7 **se caractérise par** ce que la protection de l'élément de mesure de la température (5) est situé à la mi-hauteur de la douille (3).
